# EUROPÄISCHE PATENTSCHRIFT

(11) **EP 1 123 084 B1**
(45) Veröffentlichungstag und Bekanntmachung des Hinweises auf die Patenterteilung: **29.12.2004**
(21) Anmeldenummer: 99950580.3
(22) Anmeldetag: 30.09.1999
(51) Int. Cl.: A61K 9/00

(54) **VERWENDUNG VON TESTOSTERONESTERN UND/ODER TESTOSTERON ZUR HERSTELLUNG VON BUKKAL APPLIZIERBAREN BIOADHASIVEN SYSTEMEN MIT ZEITGESTEUERTER ARZNEISTOFFWIRKUNG**
USE OF TESTOSTERONE ESTERS AND/OR TESTOSTERONE FOR PRODUCING BUCALLY APPLICABLE BIO-ADHESIVE SYSTEMS WITH TIME-RELEASED ACTIVE INGREDIENTS
UTILISATION D'ESTERS DE TESTOSTERONE ET/OU DE TESTOSTERONE POUR PRODUIRE DES SYSTEMES BIOADHESIFS D'APPLICATION BUCCALE CONTENANT DES PRINCIPES ACTIFS MEDICAMENTEUX A LIBERATION RETARDEE

(30) Priorität: 02.10.1998 DE 19847252
(43) Veröffentlichungstag der Anmeldung: 16.08.2001
(73) Patentinhaber: Schering AG, 13353 Berlin (DE)
(72) Erfinder: HÜBLER, Doris, D-07407 Schmieden (DE); KAUFMANN, Günter, D-07743 Jena (DE); OETTEL, Michael, D-07743 Jena (DE); ZIMMERMANN, Holger, D-98693 Ilmenau-Roda (DE); DITTGEN, Michael, D-99510 Apolda (DE); FRICKE, Sabine, D-07749 Jena (DE); BÖSE, Manfred, D-07745 Jena (DE); LADWIG, Ralf, D-07745 Jena (DE); CLAUSSEN, Sven, D-07743 Jena (DE); TIMPE, Carsten, D-37299 Wei enborn (DE)
(74) Vertreter: Cramer, Eva-Maria
(86) Internationale Anmeldenummer: PCT/EP1999/007254
(87) Internationale Veröffentlichungsnummer: WO 2000/019975

(56) Entgegenhaltungen:
- WO-A-97/38663
- VOORSPOELS, JODY; ET AL.: "Buccal Absorption of Testosterone and Its Esters Using a Bioadhesive Tablet in Dogs" PHARMACEUTICAL RESEARCH, Bd. 13, Nr. 8, August 1996 (1996-08), Seiten 1228-1232, XP000865566 US
- WANG, CHRISTINA; ET AL.: "Testosterone undecanoate and testosterone cyclodextrin" PHARMACOLOGY, BIOLOGY, AND CLINICAL APPLICATIONS OF ANDROGENS. PROC. INT. ANDROG. WORKSHOP, 2ND.,1995, Seiten 487-491, XP000865802

## Beschreibung

Die Erfindung betrifft die Kombination von Testosteron mit Testosteronestern (oder alleinige Verwendung von Testosteronestern) in einer bukkalen, bioadhäsiven Zubereitung, so daß durch feinabgestimmte Dosierung der Wirkstoffe und Auswahl der Ester zeitgesteuert verschiedene gewünschte Testosteron-Plasmaspiegel bei Patienten individuell, z.B. zur Wiederherstellung eines zirkadianen körpereigenen Rhythmus ein- bzw. hergestellt werden können.

Testosteron ist quantitativ und qualitativ das bedeutendste Androgen, das im Körper synthetisiert wird. Es wird hauptsächlich in den Testes, in geringer Menge in der Nebennierenrinde und bei Frauen in den Ovarien gebildet. Beim Mann ist Testosteron verantwortlich für die Entwicklung der männlichen Ausprägung während der fetalen-, neonatalen und pubertären Reifung und schließlich für den Erhalt des männlichen Phänotyps, sowie Androgenabhängiger Funktionen (z.B. Spermatogenese). Testosteron hat protein-anabole Wirkung (an Muskulatur, Knochen, Hämatopoese, Niere, Leber) [E. Mutschler, Arzneimittelwirkungen, 6. Auflage, S. 334- 337, Wissenschaftliche Verlagsgesellschaft mbH Stuttgart 1991].

Bei oraler oder parenteraler Gabe von Testosteron findet eine schnelle Absorption im Gastrointestinaltrakt, anschließend Transport über die Portalvene in die Leber mit nachfolgender hoher Metabolisierung statt, die eine kurze Plasmahalbwertszeit des Testosterons von ca. 10 min bedingt [Auterhoff, H., Knabe, J., Höltje, H.-D., Lehrbuch der Pharmazeutischen Chemie, 12. Aufl.,570 - 573, Wissenschaftliche Verlagsgesellschaft mbH Stuttgart 1991]. Zum Aufbau physiologischer Serumspiegel ist die orale Gabe von 400 mg (!) Testosteron erforderlich [S.G.Johnson, E.P. Bennet, V.G.Jensen: Therapeutic effectiveness of oral testosterone. Lancet, 2, 1974, 1473 - 1475].

Um eine Verlängerung der Testosteronwirkung zu erzielen, injiziert man Testosteronester (Testosteronpropionat, Testosteronenantat, Testosteronundecanoat) verschiedener Kettenlänge intramuskulär als ölige Lösung oder Suspension. Es ist bekannt, daß im Kontakt mit Körperflüssigkeiten des Gewebes diese Ester langsam unter Einfluß von Esterasen hydrolysieren und das pharmakologisch wirksame Testosteron freigeben. Der Einfluß der Esterart auf das Wachstum des Kapaunenkammes nach i.m. Injektion ist bereits beschrieben [Meier R. und Tschopp E.: Arch. Exptl. Pathol. Pharmacol. 226,532 (1955)].

Darüberhinaus wird Testosteronundecanoat (als Weichgelatinekapsel in Ölsäure) oral über den lymphatischen Weg appliziert [Präparat Andriol®]. über die Ölsäureeinbettung gelangt der Arzneistoff aus dem Gastrointestinaltrakt über den Ductus thoracicus in die Lymphbahn und somit in die systemische Zirkulation. Es resultieren jedoch variable Serumspiegel und gastrointestinale Nebenwirkungen. Diese Effekte können eine Dauertherapie zur Substitution erschweren [A.M. Matsumoto: Hormonal therapy of male hypogonadism. Endocrinol. Metab. Clin. North Am. 23, 1994, 857 - 875].

Andere Applikationsrouten (transdermal, nasal, sublingual, bukkal. subkutan) wurden von verschiedenen Forschergruppen untersucht [z.B.: N.A. Mazer, W.E. Heiber, J.F. Moellmer, A.W. Meikle, J.D. Stringham, S.W. Sanders, K.G.Tolman, W.D. Odell: Enhanced transdermal delivery of testosterone: a new physiological approach for androgen replacement in hypogonadal men. J.Controll.Rel. 19:347 - 362 (1992)].

Nachteile der vorgenannten Therapien sind 1. entweder ein zu kurzer, schnell abflutender Testosteronspiegel (bei oraler Gabe) oder 2. - im Fall der intramuskulären Injektion von Testosteronestern - die Unveränderbarkeit konstant eingestellter Testosteronspiegel über längere Zeiträume (Tage bis Wochen), die keine individuelle zeitliche Steuerung der Testosteronwirkung in einer Applikationsform ermöglichen. Zusätzlich kann es hier zur Downregulation der basalen Testosteron-Sekretion kommen.

Die durch den hohen First-Pass-Effekt in der Leber bedingte geringe Bioverfügbarkeit kann durch bukkale bzw. sublinguale Applikation umgangen werden. Verschiedene Studien belegen dies [z.B.: Pitha, J., Anaissie, J., Uekama, K.: g-Cyclodextrin: testosterone complex suitable for sublingual administration. J. Pharmaceutical Sciences, 76 (10) 1987, 788 - 790].

Die bukkale Applikation von Arzneistoffen ist im Stand der Technik bekannt.

EP-0371466 A betrifft eine schnell-lösliche Tablette zur raschen bukkalen Applikation u.a. von Steroiden (z.B. Estrogene, Progestine). Als Haupt-Hilfsstoffbestandteil wird ein wasserlöslicher Polyalkohol verwendet, in erster Linie Sorbitol. Vorteil ist der rasche initiale Anstieg der Arzneistoffkonzentration.

EP-0286581 A beinhaltet die transmukosale bukkale Applikation von Estrogenen (17β-Estradiol und Ethinylestradiol): Das Estrogen wird im Rahmen der Hormon-Replacement-Therapie bei postmenopausalen Frauen zur Behandlung des PMS (postmenopausales Syndrom) und Osteoporosetherapie in einer Dosierung von 50 - 100 µg/Tag (17β-Estradiol zur PMS-Therapie) bzw. 200 - 400 µg/Tag (17β-Estradiol zur Osteoporosetherapie) eingesetzt. Durch die bukkale Anwendung können therapeutische Plasmaspiegel unter Umgehung des First-Pass-Effekts erzielt werden.

WO -704342 beschreibt eine spezielle Rezeptur, die u.a. besonders für die bukkale Applikation von Estrogenen (z.B. Estradiol und Ester des Estradiols), Progestinen, Androgenen und anabolischen Steroiden geeignet sein soll: Die Formulierung enthält a) ca. 1 - 20 % eines löslichen, adhäsiven Polymers (Carbomere, partiell hydrolysiertes PVA, Polyethylenoxid, Polyacrylat, Hydroxypropylmethylcellulose, b) einen löslichen, direkttablettierbaren Hilfsstoff und c) den Wirkstoff. Das adhäsive Polymer fixiert die Rezeptur bzw. Arzneiform an der Applikationsstelle.

US-4396615 A beschreibt die Behandlung Androgen-bezogener Erkrankungen durch Gabe des Testosteron-5α-Reduktase Inhibitors 6-Methylen-Progesteron mittels topischer Formulierungen. Diese enthalten den Inhibitor und einen inerten topischen Carrier (u.a. Silikone, Methylcellulose, Hydroxymethylcellulose).

CA-2105887 A offenbart ein bio-erodierbares System zur bukkalen und vaginalen Applikation u.a. von Hormonen (Estradiol). Das System ist wasserlöslich und mucoadhärent. Es besteht aus einem festen, löslichen, lyophilisierten Schaum und dem Wirkstoff. Die Auflösungszeit beträgt mindestens 8 h. Das System haftet an der mukosalen Membran, wo es den Arzneistoff freigibt. Verwendete Polymere sind vornehmlich Gelatine, Natriumcarboxymethylcellulose, Methylcellulose etc. Vorteil des Systems ist insbesondere die lange Haftdauer.

Bezüglich einer gezielten Zeitsteuerung von Arzneimitteln ist anzumerken, daß es bei unsere heute verfügbaren Arzneimitteln nicht möglich ist, die Wirkstofffreisetzung variabel zu gestalten und eine Anpassung der Freisetzung an den individuellen Arzneistoffbedarf des Patienten vorzunehmen.

Zur Freisetzungssteuerung werden bei herkömmlichen Depotarzneimitteln vorwiegend passiv ablaufende Auflösungs-, Diffusions-, Quellungs- und Erosionsprozesse eingesetzt [Gröning, R., Arzneiformen mit elektronisch gesteuerter Freisetzung, in Pharmazeutische Technologie: Moderne Arzneiformen, S. 441. Aufl., Müller, R.H., Hildebrand, G.E. (Hrsg.), Wissenschaftliche Verlagsgesellschaft mbH Stuttgart 1998).

Die zeitgesteuerten Freisetzung von Arzneimitteln ist ebenfalls aus dem Stand der Technik bekannt.

JP-07118143 betrifft zeitkontrollierte Kapseln, die a) wasserunlöslich oder partiell wasserlöslich sind, b) aus in Wasser quellbare Substanzen (Pulver, Granulate oder Pillen z.B. aus Calciumcarboxymethylcellulose, Polyvinylpyrrolidon), c) Elemente (Tabletten) mit Wirkstoff im Zentrum der Kapseln enthalten.

Weiterer Stand der Technik betrifft Retardzubereitungen unter Polymerverwendung, wie beispielsweise EP-068446 zur Verwendung von Methylcellulose oder Carboxymethylcellulose in Mischungen zur Retardierung.

WANG, CHRISTINA; ET AL.: "Testosterone undecanoate and testosterone cyclodextrin" PHARMACOLOGY, BIOLOGY, AND CLINICAL APPLICATIONS OF ANDROGENS. PROC. INT. ANDROG. WORKSHOP, 2ND., 1995, Seiten 487-491, XP000865802 offenbart die Verwendung von oralen oder sublingualen Testosteron enthaltenden Arzneimittel, insbesondere Testosteroncyclodextrin oder Testosteronundecanoat.

Aufgabe der Erfindung ist es, die Vorteile der bukkalen Applikation von Steroiden mit hohem First-Pass-Effekt und geringer Bioverfügbarkeit, speziell von Testosteron und seinen Estern, mit der Nutzung der unterschiedlichen Pharmakokinetik der verschiedenen Testosteronester (je nach Kettenlänge ) derart zu nutzen, daß durch sorgfältige Auswahl geeigneter Dosierungrn und Ester das jeweils gewünschte Arzneistoffprofil eingestellt werden kann.

Die Aufgabe wird durch die Verwendung von Testosteronestern mit 1 bis 20 C-Atomen im Carbonsäurerest oder Mischungen aus zwei oder mehreren Testosteronestern mit unterschiedlichen Carbonsäureestern und/oder Testosteron zur Herstellung von bukkal applizierbaren bioadhäsiven Systemen mit zeitlich gesteuerter Wirkstofffreisetzung zur Behandlung von Erkrankungen, welche mit einem veränderten Testosteronspiegel einhergehen, wobei die bukkal zu applizierenden bioadhäsiven Systeme erhältlich sind, indem man die Wirkstoffe zunächst getrennt oder gemeinsam in einem Sprühtrocknungsverfahren amorph in organische Polymere einbettet, gelöst.

Bevorzugt ist es, daß Testosteronester verwendet werden, bei denen die Kohlenstoffgerüste der Carbonsäurereste gradkettig, verzweigt, alicyclisch, gesättigt und/oder ungesättigt mit bis zu fünf Doppel- und/oder Dreifachbindungen sind.

Bevorzugt ist es ferner, daß das Verhältnis von Testosteron zu Testosteronester 1 : 100 bis 1 : 1, besonders bevorzugt 1 : 10 bis 1 :1,5 beträgt.

Ganz besonders bevorzugt ist es dabei, daß man die amorphen Testosteron-Sprühprodukte zusammen mit anderen Hilfsstoffen, Bindemitteln, Füllmitteln, Gleitmitteln, bioadhäsiven Polymeren, Tensiden, Zerfallbeschleunigern mischt und zu Ein- oder Mehrschichttabletten verpreßt.

Bevorzugt ist die erfindungsgemäße Verwendung zur gezielten Einstellung therapeutischer und/oder zirkadianer Rhythmen der Testosteronspiegel.

Überraschenderweise wurde gefunden, daß die Kettenlänge des Testosteronesters nicht nur die Löslichkeit bestimmt, sondern, wie experimentell gezeigt werden konnte, offenbar auch die Esterspaltungskinetik im Blut oder Gewebeflüssigkeiten durch entsprechende Hydrolasen. Besonders hervorzuheben ist beim Einsatz der Testosteronester die Verwendung von Sprüheinbettungstechniken in organische Polymere (Polyvinylpyrrolidon, Hydroxypropylmethylcellulose, feste Polyaethylenglycole), um eine entsprechende verbesserte Löslichkeit in der Mundhöhle zu erzielen. Dies ist besonders wichtig vor dem Hintergrund geringer Speichelvolumina (ca. 1 - 1,5 ml), die dort im Durchschnitt zur Lösung der schwerlöslichen Ester zur Verfügung stehen. In der Publikation von Jody Voorspoels [Vorspoels, J., Remon, J.-P., Eechaute, W.E., De Sy, W., Buccal Absorption of Testosterone and Its Esters Using a Bioadhesive Tablet in Dogs, Pharmaceutical Research, Vol. 13, No. 8, 1996, 1228 - 1232) zeigten die an 6 kastrierten Beagle-Hunden untersuchten Testosteronester (Testosteronacetat, Testosteronpropionat, Testosteronenantat, Testosterondecanoat) trotz der höheren Lipophilie keine höhere Bioverfügbarkeit als Testosteron. Da die Ester nur durch trockenes Mischen direkt verpreßt wurden, ohne daß eine spezielle Präformulierungstechnologie (Amorphisierung durch Sprüheinbettung) zur Anwendung kam, beruhen diese schlechteren Ergebnisse vermutlich auf der teilweise extrem geringen Löslichkeit der Ester im kristallinen Zustand.

Es konnte gezeigt werden, daß durch die bukkale Applikation von Testosteron in Kombination mit Testosteronestern verschiedener Kettenlänge unterschiedliche Blutspiegelmuster bzw. -rhythmen (i.S. einer zirkadianen Rhythmik) eingestellt werden können. Bei der Auswahl der Testosteronester kann die Auswahl gezielt aus drei Gruppen vorgenommen werden: 1. Ester kürzerer Kettenlänge (z.B. Testosteronacetat oder -propionat), 2.Ester mittlerer Kettenlänge (z.B. Testosteronenanthat, -cipionat,- cyclohexancarboxylat) und 3. Ester höherer Kettenlänge (z.B. Tetsosteronundecanoat, -bucyclat).

Erfindungsgemäß wird die Aufgabe dadurch gelöst, daß Testosteron bzw. der jeweilige Testosteronester in Ethanol zusammen mit dem Polymer (z.B. Polyvinylpyrrolidon oder Hydroxypropylmethylcellulose) gelöst und zu einer amorphen Sprüheinbettung in einer Sprühtrocknungsanlage weiterverarbeitet wird. Dabei ist es möglich, daß 1. die genannten Wirkstoffe getrennt voneinander oder 2. zusammen in einem Arbeitsschritt miteinander amorph eingebettet werden.

Die feinkörnige Sprüheinbettung wird dann mit anderen Hilfsstoffen zur Herstellung bioadhäsiver Bukkaltabletten (Bindemittel: Polyvinylpyrrolidon, Celluloseether; Füllmittel: Cellactose®, Mannitol, Sorbitol, Lactose, Gleitmittel: Magnesiumstearat, hydrierte Pflanzenfette, bioadhäsive Polymere: Polyacrylate [Carbopole®, Natriumcarboxymethylcellulose] und ggf. weitere Hilfsstoffe, wie Tenside, Zerfallsbeschleuniger) trocken gemischt und zu Bukkaltabletten gepreßt, die auch schichtartig aufgebaut sein können (Wirkstoffschicht, bioadhäsive Klebeschicht; uni- oder multidirektionale Freisetzung).

Ausgehend von Blutspiegeluntersuchungen mit den Monosubstanzen können über die Wahl der beiden Parameter
- Dosierung der Wirkstoffkomponente
- Wahl des Esters bzw. der Kettenlänge an C-17 entsprechende Release-Muster eingestellt werden.

Als vorteilhaft hat sich beispielsweise die Kombination des kurzwirksamen Testosterons zusammen mit dem Testosteronundecanoat (C-11-Kette) mit längerer Halbwertszeit ergeben (s. Ausführungsbeispiele).

Bevorzugt ist hierbei ein Verhältnis der Wirkstoffe zueinander, wobei das Verhältnis Testosteron: Σ Testosteronester: 1 : 100 bis 1 : 1, besonders bevorzugt 1 : 10 bis 1 : 1,5 beträgt.

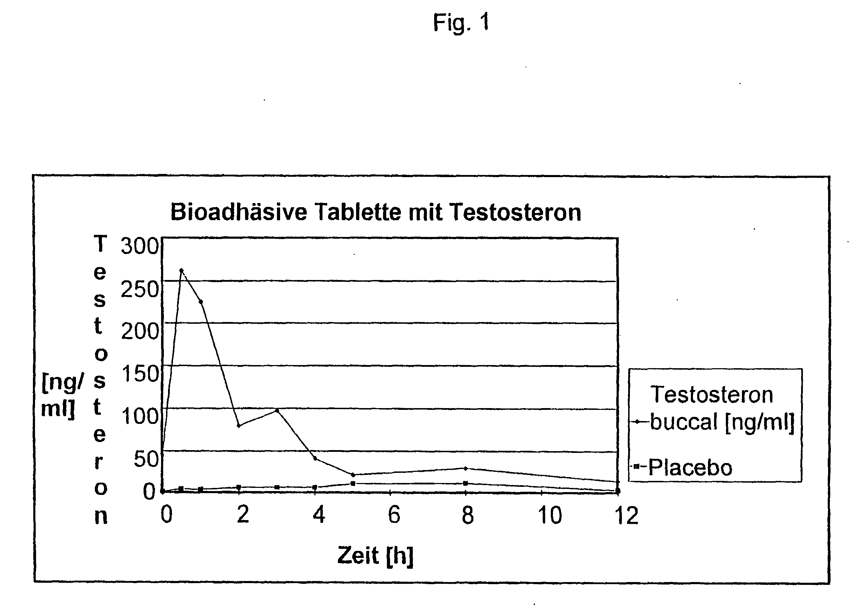

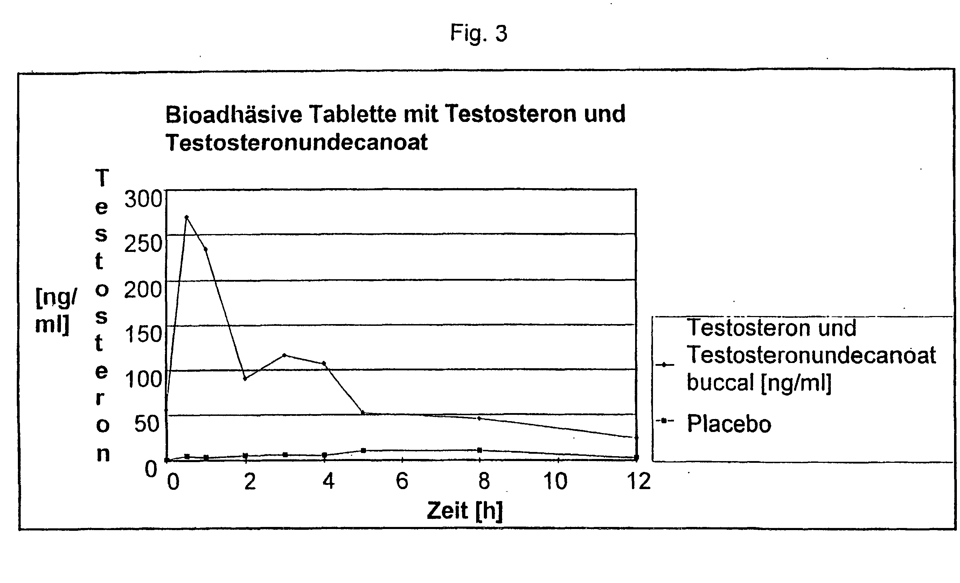

Durch geschicktes Kombinieren von Testosteron und Testosteronestern ist die Einstellung von Blutspiegelverläufen möglich, die die körpereigene Rhythmizität endogener Testosteronspiegel abzubilden bzw. zu simulieren in der Lage sind: So kann beispielsweise die Wirkungsdauer einer bioadhäsiven Bukkaltablette mit Testosteron durch die Kombination mit Testosteronundecanoat verlängert werden (vgl. Fig.1 und Fig. 3): Blutspiegelwerte > 100 ng/ml werden im Anwendungsbeispiel (äquimolare Kombination) am weiblichen Hund von ca. 2 h auf über 4 h verlängert; darüberhinaus läßt sich ein ausgeprägter (kleinerer) 2. Puls mit einem Maximum nach ca. 3 h erkennen. Nach 8 h sind die Testosteronwerte mit fast 50 ng/ml immer noch doppelt so hoch im Vergleich zur Gabe der Monosubstanz.

Im wesentlichen wird der eingestellte Blutspiegelverlauf an Testosteron durch zwei Parameter gesteuert:
- die Kettenlänge und räumliche Struktur der Esterkette an C-17: Diese bestimmt einerseits die Lipophilie und damit die Löslichkeit, andererseits in ausgeprägter Weise die Esterspaltungs- bzw. Hydrolysegeschwindigkeit (Wechselwirkungen der Esterseitenkette mit dem aktiven Zentrum der Hydrolasen). So werden Ester höherer Kettenlänge langsamer gespalten als solche mit mittlerer bzw. kürzerer Länge. In diesem Sinne flutet das dadurch freigesetzte Testosteron langsamer an als bei Gabe reinen Testosterons; der Peak erscheint später. Über die Kettenlänge besteht somit die Möglichkeit einer mehr oder minder gezielten Zeitsteuerung der arzneilichen Wirkung;
- die Dosierung von Testosteron bzw. Testosteronester über die direkte Proportionalität der AUC in Relation zur applizierten Dosis

Dies könnte eine Bedeutung für die Hormonsubstitution (Hormone Replacement Therapy) bei älteren Männern mit partiellem Androgendefizit (sog. PADAM-Patienten) haben, indem mangelnde Plasmaspiegel entsprechend therapeutisch korrigiert werden.

Die folgenden Ausführungsbeispiele erläutern die Erfindung in nicht-beschränkender Weise:

### Beispiel 1:

### Bioadhäsive Tablette mit Testosteron

### Rezeptur

| ***Bestandteil*** | ***Masse*/*Tablette*** |
|---|---|
| ***Wirkstoffschicht*** | |
| *Testosteron-Präformulierung (20%)* | *50,00* |
| *Mannitol* | *43,90* |
| *Cellactose 80* | *29,50* |
| *Carmellose-Natrium* | *1,20* |
| *Magnesiumstearat* | *1,50* |
| *Talkum* | *4,30* |

| ***Klebeschicht*** | |
|---|---|
| *Mannitol* | *17,40* |
| *Cellactose 80* | *34,82* |
| *Carmellose-Natrium* | *10,18* |
| *Magnesiumstearat* | *0, 65* |
| *Eisenoxidrot* | *0,05* |
| *Talkum* | *1,90* |

### Herstellung:

### Herstellung der Präformulierung:

Die Testosteron-Präformulierung (20 %) wird durch Sprühtrocknung hergestellt. Dabei wird der Wirkstoff zusammen mit dem Polymer (Polyvinylpyrrolidon, Hydroxypropylmethylcellulose z.B.) und ggf. einem weiteren Hilfsstoff (Träger oder antistatisch wirkender Hilfsstoff) in einem geeigneten Lösungsmittel gelöst bzw. suspendiert. Die durch ständiges Rühren homogenisierte Suspension wird in einer Sprühtrocknungsanlage zu einem feinkörnigen Pulver sprühgetrocknet.

### Herstellung der Wirkstoffschicht

Diese Präformulierung wird zusammen mit den anderen Hilfsstoffen der Wirkstoffschicht (Mannitol, Cellactose 80) in einem geeigneten Mischer (Typ: Kubusmischer, Turbulamischer, Taumelmischer etc.) ca. 20 min und dann unter Zufügen der äußeren Phase (Carmellose-Natrium, Magnesiumstearat, Talkum) nochmals 5 min gemischt.

### Herstellung der Klebeschicht

Eisenoxidrot und Talkum werden in einer Kugelmühle innig verrieben. Anschließend mischt man ca. 20 min in einem geeigneten Mischer (Typ: Kubusmischer, Turbula-mischer, Taumelmischer etc.) Mannitol und Cellactose 80, gibt die Farbverreibung sowie Carmellose-Natrium und Magnesiumstearat als äußere Phase hinzu und mischt nochmals 5 min.

Wirkstoff- und Klebeschicht werden anschließend auf einer geeigneten Tablettenpresse entsprechend der Massenrelation zu Zweischichttabletten verpreßt.

Fig. 1 zeigt den Blutspiegelverlauf einer Formulierung nach Beispiel 1.

### Beispiel 2:

### Bioadhäsive Tablette mit Testosteronundecanoat

### Rezeptur

| ***Bestandteil*** | ***Masse*/*Tablette*** |
|---|---|
| ***Wirkstoffschicht*** | |
| *Testosteronundecanoat-Präformulierung (20%)* | *50,00* |
| *Mannitol* | *43,90* |
| *Cellactose 80* | *29,50* |
| *Carmellose-Natrium* | *1,20* |
| Magnesiumstearat | *1,50* |
| *Talkum* | *4,30* |

| ***Klebeschicht*** | |
|---|---|
| *Mannitol* | *17,40* |
| *Cellactose 80* | *34,82* |
| *Carmellose-Natrium* | *10,18* |
| *Magnesiumstearat* | *0,65* |
| *Eisenoxidrot* | *0,05* |
| *Talkum* | *1,90* |

### Herstellung:

### Herstellung der Präformulierung:

Die Testosteronundecanoat-Präformulierung (20 %) wird durch Sprühtrocknung hergestellt. Dabei wird der Wirkstoff zusammen mit dem Polymer (Polyvinylpyrrolidon, Hydroxypropylmethylcellulose z.B.) und ggf. einem weiteren Hilfsstoff (Träger oder antistatisch wirkender Hilfsstoff) in einem geeigneten Lösungsmittel gelöst bzw. suspendiert. Die durch ständiges Rühren homogenisierte Suspension wird in einer Sprühtrocknungsanlage zu einem feinkörnigen Pulver sprühgetrocknet.

### Herstellung der Wirkstoffschicht

Diese Präformulierung wird zusammen mit den anderen Hilfsstoffen der Wirkstoffschicht (Mannitol, Cellactose 80) in einem geeigneten Mischer (Typ: Kubusmischer, Turbulamischer, Taumelmischer etc.) ca. 20 min und dann unter Zufügen der äußeren Phase (Carmellose-Natrium, Magnesiumstearat, Talkum) nochmals 5 min gemischt.

### Herstellung der Klebeschicht

Eisenoxidrot und Talkum werden in einer Kugelmühle innig verrieben. Anschließend mischt man ca. 20 min in einem geeigneten Mischer (Typ: Kubusmischer, Turbulamischer, Taumelmischer etc.) Mannitol und Cellactose 80, gibt die Farbverreibung sowie Carmellose-Natrium und Magnesiumstearat als äußere Phase hinzu und mischt nochmals 5 min.

Wirkstoff- und Klebeschicht werden anschließend auf einer geeigneten Tablettenpresse entsprechend der Massenrelation zu Zweischichttabletten verpreßt.

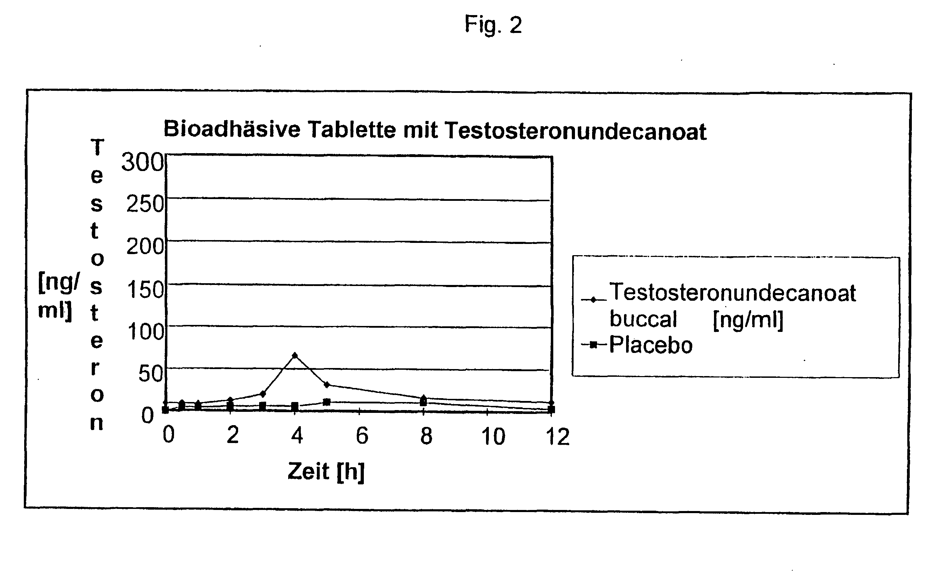

Fig. 2 zeigt den Blutspiegelverlauf einer Formulierung nach Beispiel 2.

### Beispiel 3:

### Bioadhäsive Tablette mit Testosteron und Testosteronundecanoat

### Rezeptur

| ***Bestandteil*** | ***Masse*/*Tablette*** |
|---|---|
| ***Wirkstoffschicht*** | |
| *Testosteron-Testosteronundecanoat-Präformulierung (20%,20%)* | *50,00* |
| *Mannitol* | *43,90* |
| *Cellactose 80* | *29,50* |
| *Carmellose-Natrium* | *1,20* |
| *Magnesiumstearat* | *1,50* |
| *Talkum* | *4,30* |

| ***Klebeschicht*** | |
|---|---|
| *Mannitol* | *17,40* |
| *Cellactose 80* | *34,82* |
| *Carmellose-Natrium* | *10,18* |
| *Magnesiumstearat* | *0,65* |
| *Eisenoxidrot* | *0,05* |
| *Talkum* | *1,90* |

### Herstellung:

### Herstellung der Präformulierung:

Die Testosteron/Testosteronundecanoat-Präformulierung (20 %) wird durch Sprühtrocknung hergestellt. Dabei werden beide Wirkstoffe zusammen mit dem Polymer (Polyvinylpyrrolidon, Hydroxypropylmethylcellulose z.B.) und ggf. einem weiteren Hilfsstoff (Träger oder antistatisch wirkender Hilfsstoff) in einem geeigneten Lösungsmittel gelöst bzw. suspendiert. Die durch ständiges Rühren homogenisierte Suspension kann in einer Sprühtrocknungsanlage zu einem feinkörnigen Pulver sprühgetrocknet werden.

### Herstellung der Wirkstoffschicht

Diese Präformulierung wird zusammen mit den anderen Hilfsstoffen der Wirkstoffschicht (Mannitol, Cellactose 80) in einem geeigneten Mischer (Typ: Kubusmischer, Turbula-mischer, Taumelmischer etc.) ca. 20 min und dann unter Zufügen der äußeren Phase (Carmellose-Natrium, Magnesiumstearat, Talkum) nochmals 5 min gemischt.

### Herstellung der Klebeschicht

Eisenoxidrot und Talkum werden in einer Kugelmühle innig verrieben. Anschließend mischt man ca. 20 min in einem geeigneten Mischer (Typ: Kubusmischer, Turbula-mischer, Taumelmischer etc.) Mannitol und Cellactose 80, gibt die Farbverreibung sowie Carmellose-Natrium und Magnesiumstearat als äußere Phase hinzu und mischt nochmals 5 min.

Wirkstoff- und Klebeschicht werden anschließend auf einer geeigneten Tablettenpresse entsprechend der Massenrelation zu Zweischichttabletten verpreßt.

Fig. 3 zeigt den Blutspiegelverlauf einer Formulierung nach Beispiel 3.

### Beispiel 4:

### Bioadhäsive Tablette mit Testosteron, Testosteronenanthat)

### Rezeptur

| ***Bestandteil*** | ***Masse*/*Tablette*** |
|---|---|
| ***Wirkstoffschicht*** | |
| *Testosteron-Testosteronenanthat-Präformulierung (20%,20%)* | *50,00* |
| *Mannitol* | *58,60* |
| *Emcompress* | *14,00* |
| *Carbopol® 974* | *2,00* |
| *Magnesiumstearat* | *1,50* |
| *Talkum* | *4, 30* |

| ***Klebeschicht*** | |
|---|---|
| *Mannitol* | *17,40* |
| *Emcompress* | *34, 82* |
| *Carbopol® 974* | *13,00* |
| *Magnesiumstearat* | *0, 65* |
| *Eisenoxidrot* | *0,05* |
| *Talkum* | *1,90* |

### Herstellung:

### Herstellung der Präformulierung:

Die Testosteron/Testosteronenanthat-Präformulierung (20%) wird durch Sprühtrocknung hergestellt. Dabei werden beide Wirkstoffe zusammen mit dem Polymer (Polyvinylpyrrolidon, Hydroxypropylmethylcellulose z.B.) und ggf. einem weiteren Hilfsstoff (Träger oder antistatisch wirkender Hilfsstoff) in einem geeigneten Lösungsmittel gelöst bzw. suspendiert. Die durch ständiges Rühren homogenisierte Suspension wird in einer Sprühtrocknungsanlage zu einem feinkörnigen Pulver sprühgetrocknet.

### Herstellung der Wirkstoffschicht

Diese Präformulierung wird zusammen mit den anderen Hilfsstoffen der Wirkstoffschicht (Mannitol, Emcompress) in einem geeigneten Mischer (Typ: Kubusmischer, Turbulamischer, Taumelmischer etc.) ca. 20 min und dann unter Zufügen der äußeren Phase (Carbopol® 974, Magnesiumstearat, Talkum) nochmals 5 min gemischt.

### Herstellung der Klebeschicht

Eisenoxidrot und Talkum werden beispielsweise in einer Kugelmühle innig verrieben. Anschließend mischt man ca. 20 min in einem geeigneten Mischer (Typ: Kubusmischer, Turbulamischer, Taumelmischer etc.) Mannitol und Emcompress, gibt die Farbverreibung sowie Carbopol® 974 und Magnesiumstearat als äußere Phase hinzu und mischt nochmals 5 min.

Wirkstoff- und Klebeschicht werden anschließend auf einer geeigneten Tablettenpresse entsprechend der Massenrelation zu Zweischichttabletten verpreßt.

### Beispiel 5:

### Bioadhäsive Tablette mit Testosteronpropionat und Testosterondecanonat

### Rezeptur

| ***Bestandteil*** | ***Masse*/*Tablette*** |
|---|---|
| ***Wirkstoffschicht*** | |
| *Testosteronpropionat Testosterondecanoat-Präformulierung (20%,20%)* | *50,00* |
| *Mannitol* | *58,60* |
| *Emcompress* | *14,00* |
| *Carbopol® 974* | *2,00* |
| *Magnesiumstearat* | *1,50* |
| *Talkum* | *4,30* |

| ***Klebeschicht*** | |
|---|---|
| *Mannitol* | *17,40* |
| *Emcompress* | *34,82* |
| *Carbopol® 974* | *13,00* |
| *Magnesiumstearat* | *0,65* |
| *Eisenoxidrot* | *0,05* |
| *Talkum* | *1,90* |

### Herstellung:

### Herstellung der Präformulierung:

Die Testosteronpropionat/Testosterondecanoat-Präformulierung (20 %) wird durch Sprühtrocknung hergestellt. Dabei werden beide Wirkstoffe zusammen mit dem Polymer (Polyvinylpyrrolidon, Hydroxypropylmethylcellulose z.B.) und ggf. einem weiteren Hilfsstoff (Träger oder antistatisch wirkender Hilfsstoff) in einem geeigneten Lösungsmittel gelöst bzw. suspendiert. Die durch ständiges Rühren homogenisierte Suspension wird in einer Sprühtrocknungsanlage zu einem feinkörnigen Pulver sprühgetrocknet.

### Herstellung der Wirkstoffschicht

Diese Präformulierung wird zusammen mit den anderen Hilfsstoffen der Wirkstoffschicht (Mannitol, Emcompress) in einem geeigneten Mischer (Typ: Kubusmischer, Turbulamischer, Taumelmischer etc.) ca. 20 min und dann unter Zufügen der äußeren Phase (Carbopol® 974, Magnesiumstearat, Talkum) nochmals 5 min gemischt.

### Herstellung der Klebeschicht

Eisenoxidrot und Talkum werden in einer Kugelmühle innig verrieben. Anschließend mischt man ca. 20 min in einem geeigneten Mischer (Typ: Kubusmischer, Turbulamischer, Taumelmischer etc.) Mannitol und Emcompress, gibt die Farbverreibung sowie Carbopol® 974 und Magnesiumstearat als äußere Phase hinzu und mischt nochmals 5 min.

Wirkstoff- und Klebeschicht werden anschließend auf einer geeigneten Tablettenpresse entsprechend der Massenrelation zu Zweischichttabletten verpreßt.

## Patentansprüche

1. Verwendung von Testosteronestern mit 1 bis 20 C-Atomen im Carbonsäurerest oder Mischungen aus zwei oder mehreren Testosteronestern mit unterschiedlichen Carbonsäureestern und/oder Testosteron zur Herstellung von bukkal applizierbaren bioadhäsiven Systemen mit zeitlich gesteuerter Wirkstofffreisetzung zur Behandlung von Erkrankungen, welche mit einem veränderten Testosteronspiegel einhergehen,
**dadurch gekennzeichnet, daß** die bukkal zu applizierenden bioadhäsiven Systeme erhältlich sind, indem man die Wirkstoffe zunächst getrennt oder gemeinsam in einem Sprühtrocknungsverfahren amorph in organische Polymere einbettet.

2. Verwendung gemäß Anspruch 1, **dadurch gekennzeichnet, daß** die Kohlenstoffgerüste der Carbonsäurereste gradkettig, verzweigt, alicyclisch, gesättigt und/oder ungesättigt mit bis zu fünf Doppel- und/oder Dreifachbindungen sind.

3. Verwendung gemäß Anspruch 1 oder 2, **dadurch gekennzeichnet, daß** das Verhältnis von Testosteron zu Testosteronester 1 : 100 bis 1 : 1, vorzugsweise 1 : 10 bis 1 :1,5 beträgt.

4. Verwendung gemäß einem der Ansprüche 1 bis 3, **dadurch gekennzeichnet, daß** man die amorphen Testosteron-Sprühprodukte zusammen mit anderen Hilfsstoffen, Bindemitteln, Füllmitteln, Gleitmitteln, bioadhäsiven Polymeren, Tensiden, Zerfallbeschleunigern mischt und zu Ein- oder Mehrschichttabletten verpreßt.

5. Verwendung gemäß einem der Ansprüche 1 bis 4 zur gezielten Einstellung therapeutischer und/oder zirkadianer Rhytmen der Testosteronspiegel.

## Claims

1. Use of testosterone esters having 1 to 20 C atoms in the carboxylic acid residue or mixture of two or more testosterone esters with different carboxylic esters and/or testosterone for producing bioadhesive systems which can be administered buccally and have time-controlled release of active ingredient for the treatment of disorders which are associated with an altered testosterone level, **characterized in that** the bioadhesive systems to be administered buccally are obtainable by embedding the active ingredients initially separately or together amorphously in organic polymers in a spray-drying process.

2. Use according to Claim 1, **characterized in that** the carbon frameworks of the carboxylic acid residues are straight-chain, branched, alicyclic, saturated and/or unsaturated with up to five double and/or triple bonds.

3. Use according to Claim 1 or 2, **characterized in that** the ratio of testosterone to testosterone ester is from 1:100 to 1:1, preferably 1:10 to 1:1.5.

4. Use according to any of Claims 1 to 3, **characterized in that** the amorphous testosterone spray products are mixed together with other excipients, binders, fillers, lubricants, bioadhesive polymers, surfactants, disintegration promoters and compressed to monolayer or multilayer tablets.

5. Use according to any of Claims 1 to 4 for targeted adjustment of therapeutic and/or circadian rhythms of the testosterone levels.

## Revendications

1. Utilisation d'esters de testostérone ayant de 1 à 20 atomes de carbone dans le reste acide carboxylique ou de mélanges de deux ou plus de deux esters de testostérone avec des esters d'acides carboxyliques différents et/ou de la testostérone pour la fabrication de systèmes bioadhésifs pouvant être administrés par voie gingivo-jugale, à libération programmée dans le temps de la substance active, pour le traitement de maladies qui s'accompagnent d'un taux modifié de testostérone,
**caractérisée en ce qu'**il est possible d'obtenir les systèmes bioadhésifs à administrer par voie gingivo-jugale en incorporant d'abord les substances actives, séparément ou ensemble, dans un procédé de séchage par atomisation, à l'état amorphe dans des solvants organiques.

2. Utilisation selon la revendication 1, **caractérisée en ce que** les squelettes hydrocarbonés des restes acide carboxylique sont à chaîne droite, ramifiés, alicycliques, saturés et/ou insaturés à jusqu'à cinq doubles et/ou triples liaisons.

3. Utilisation selon la revendication 1 ou 2, **caractérisée en ce que** le rapport de la testostérone à l'ester de testostérone va de 1:100 à 1:1, de préférence de 1:10 à 1:1,5.

4. Utilisation selon l'une quelconque des revendications 1 à 3, **caractérisée en ce qu'**on mélange les produits pulvérisés de testostérone amorphes avec d'autres adjuvants, liants, excipients, lubrifiants, polymères bioadhésifs, tensioactifs, désintégrants et on les presse en comprimés mono- ou multicouches.

5. Utilisation selon l'une quelconque des revendications 1 à 4, pour l'établissement ciblé de rythmes thérapeutiques et/ou circadiens des taux de testostérone.
